# EUROPEAN PATENT SPECIFICATION

(11) **EP 1 805 183 B1**
(45) Date of publication and mention of the grant of the patent: **29.12.2010**
(21) Application number: 05801524.9
(22) Date of filing: 18.10.2005
(51) Int. Cl.: C07D 487/08, A61P 25/00, A61K 31/33

(54) **NOVEL DIAZABICYCLIC ARYL DERIVATIVES AND THEIR MEDICAL USE**
NEUE DIAZABIZYKLISCHE ARYLDERIVATE UND MEDIZINISCHE VERWENDUNG DAFÜR
NOUVEAUX DERIVES ARYLE DIAZABICYCLIQUES ET LEUR UTILISATION MEDICALE

(30) Priority: 20.10.2004 DK 200401603; 21.10.2004 US 620327 P
(43) Date of publication of application: 11.07.2007
(73) Proprietor: NeuroSearch A/S, 2750 Ballerup (DK)
(72) Inventor: PETERS, Dan, 2750 Ballerup (DK); TIMMERMANN, Daniel B., 2750 Ballerup (DK); OLSEN, Gunnar M., 2750 Ballerup (DK); NIELSEN, Elsebet Østergaard, 2750 Ballerup (DK); JORGENSEN, Tino Dyhring, 2750 Ballerup (DK)
(74) Representative: Abildgren, Michael Padkjaer
(86) International application number: PCT/EP2005/055333
(87) International publication number: WO 2006/045716

(56) References cited:
- US-A1- 2002 037 893
- US-A1- 2003 225 268
- L. TOMA, P. QUADRELLI, W. BRUNNELLE, D. ANDERSON, M. MEYER, G. CIGNARELLA, A. GELAIN AND D. BARLOCCO: "6-Chloropyridazin-3-yl derivatives active as nicotinic agents: synthesis, binding and modeling studies" JOURNAL OF MEDICINAL CHEMISTRY, vol. 45, 2002, pages 4011-4017, XP002356220

## Description

### TECHNICAL FIELD

This invention relates to novel diazabicyclic aryl derivatives, which are found to be cholinergic ligands at the nicotinic acetylcholine receptors and modulators of the monoamine receptors and transporters. Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

### BACKGROUND ART

The endogenous cholinergic neurotransmitter, acetylcholine, exert its biological effect via two types of cholinergic receptors, the muscarinic Acetyl Choline Receptors (mAChR) and the nicotinic Acetyl Choline Receptors (nAChR).

As it is well established that muscarinic acetylcholine receptors dominate quantitatively over nicotinic acetylcholine receptors in the brain area important to memory and cognition, and much research aimed at the development of agents for the treatment of memory related disorders have focused on the synthesis of muscarinic acetylcholine receptor modulators.

Recently, however, an interest in the development of nAChR modulators has emerged. Several diseases are associated with degeneration of the cholinergic system i.e. senile dementia of the Alzheimer type, vascular dementia and cognitive impairment due to the organic brain damage disease related directly to alcoholism. Indeed several CNS disorders can be attributed to a cholinergic deficiency, a dopaminergic deficiency, an adrenergic deficiency or a serotonergic deficiency.

US 2003/225268 describes certain 3,8-diazabicyclo[3.2.1]octane derivatives capable of binding to nicotinic acetylcholine receptors.

### SUMMARY OF THE INVENTION

The present invention is devoted to the provision novel modulators of the nicotinic and/or of the monoamine receptors, which modulators are useful for the treatment of diseases or disorders related to the cholinergic receptors, and in particular the nicotinic acetylcholine receptor (nAChR), the serotonin receptor (5-HTR), the dopamine receptor (DAR) and the norepinephrine receptor (NER), and of the biogenic amine transporters for serotonin (5-HT), dopamine (DA) and norepinephrine (NE).

Due to their pharmacological profile the compounds of the invention may be useful for the treatment of diseases or disorders as diverse as those related to the cholinergic system of the central nervous system (CNS), the peripheral nervous system (PNS), diseases or disorders related to smooth muscle contraction, endocrine diseases or disorders, diseases or disorders related to neuro-degeneration, diseases or disorders related to inflammation, pain, and withdrawal symptoms caused by the termination of abuse of chemical substances.

The compounds of the invention may also be useful as diagnostic tools or monitoring agents in various diagnostic methods, and in particular for *in vivo* receptor imaging (neuroimaging), and they may be used in labelled or unlabelled form.

In its first aspect the invention provides novel diazabicyclic aryl derivatives of Formula I 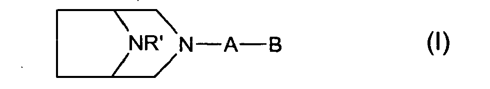 any of its enantiomers or any mixture of its enantiomers, or a pharmaceutically acceptable salt thereof, wherein
R' represents hydrogen or C₁₋₆-alkyl;
A represents an aromatic monocyclic group selected from phenyl, furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl and triazinyl; and
B represents an aromatic monocyclic or bicyclic carbocyclic or heterocyclic group, which carbocyclic or heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In its second aspect the invention provides pharmaceutical compositions comprising a therapeutically effective amount of the diazabicyclic aryl derivative of the invention, or a pharmaceutically-acceptable addition salt thereof, together with at least one pharmaceutically-acceptable carrier or diluent.

In a further aspect the invention relates to the use of the diazabicyclic aryl derivative of the invention, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

Other objects of the invention will be apparent to the person skilled in the art from the following detailed description and examples.

### DETAILED DISCLOSURE OF THE INVENTION

### Diazabicyclic Aryl Derivatives

In a first aspect novel 3,8-diaza-bicyclo[3.2.1]octane aryl derivatives are provided. The diazabicyclic aryl derivatives of the invention may be represented by the general Formula I 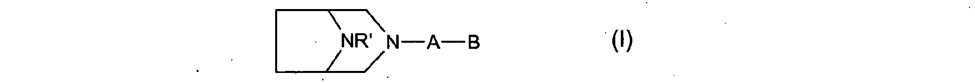 any of its enantiomers or any mixture of its enantiomers, or a pharmaceutically acceptable salt thereof, wherein
R' represents hydrogen or C₁₋₆-alkyl ;
A represents an aromatic monocyclic group selected from phenyl, furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl and triazinyl; and
B represents an aromatic monocyclic or bicyclic carbocyclic or heterocyclic group, which carbocyclic or heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In another preferred embodiment of the invention R' represents hydrogen or C₁₋₆-alkyl.

In a more preferred embodiment R' represents hydrogen.

In an even more preferred embodiment R' represents C₁₋₆-alkyl.

In a still more preferred embodiment R' represents methyl or ethyl.

In a third preferred embodiment of the invention A represents an aromatic monocyclic group selected from phenyl, furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl and triazinyl.

In a more preferred embodiment A represents an aromatic heterocyclic group selected from furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl and triazinyl.

In an even more preferred embodiment A represents oxazolyl, thiazolyl, oxadiazolyl, thiadiazolyl, pyridinyl, pyrimidinyl or pyridazinyl.

In a still more preferred embodiment A represents oxazolyl, thiazolyl, oxadiazolyl, thiadiazolyl, pyridinyl, pyrimidinyl, pyrazinyl or pyridazinyl.

In a yet more preferred embodiment A oxadiazolyl, thiadiazolyl, or pyridazinyl.

In a still further preferred embodiment A represents oxadiazolyl, in particular 1,3,4-oxadiazolyl.

In a still further preferred embodiment A represents thiadiazolyl, in particular 1,3,4-thiadiazolyl.

In a still further preferred embodiment A represents pyridinyl, in particular pyridin-2,5-diyl or pyridin-2,6-diyl.

In a still further preferred embodiment A represents pyridazinyl, in particular pyridazin-3,6-diyl.

In a still further preferred embodiment A represents pyrimidinyl, in particular pyrimidin-2,4-diyl or pyrimidin-2,5-diyl.

In a still further preferred embodiment A represents pyrazinyl, in particular pyrazin-2,5-diyl.

In a still further preferred embodiment A represents thiazolyl, in particular thiazol-2,5-diyl.

In a fourth preferred embodiment of the invention B represents an aromatic monocyclic or bicyclic carbocyclic or heterocyclic group, which carbocyclic or heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In a more preferred embodiment B represents an aromatic monocyclic or bicyclic carbocyclic or heterocyclic group, which carbocyclic or heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, amino and nitro.

In an even more preferred embodiment B represents an aromatic monocyclic heterocyclic group selected from the group consisting of furanyl, thienyl and pyridinyl, which heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In a yet more preferred embodiment B represents an aromatic monocyclic heterocyclic group, is selected from the group consisting of furanyl, thienyl and pyridinyl.

In a still further preferred embodiment B represents furanyl, in particular furan-2-yl or furan-3-yl.

In a still further preferred embodiment B represents thienyl, in particular thien-2-yl or thien-3-yl.

In a still further preferred embodiment B represents pyridinyl, in particular pyridin-2-yl or pyridin-3-yl.

In a fifth preferred embodiment B represents phenyl or naphthyl, which carbocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In a more preferred embodiment B represents phenyl or naphthyl, which carbocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, amino and nitro.

In an even more preferred embodiment B represents phenyl or naphthyl, which carbocyclic group is optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In a yet more preferred embodiment B represents phenyl, which carbocyclic group is optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl halo, trihalo-C₁₋₆-alkyl, trihalo-C₁₋₆-alkoxy, cyano, amino and nitro.

In a still more preferred embodiment B represents phenyl or naphthyl, which phenyl or naphthyl group is optionally substituted with hydroxy, C₁₋₆-alkoxy, cyano-C₁₋₆-alkyl, halo, trihalo-C₁₋₆-alkyl, trihalo-C₁₋₆-alkoxy, cyano, nitro, amino or C₁₋₆-alkyl-carbonyl-amino.

In a further preferred embodiment B represents phenyl or naphthyl, which phenyl or naphthyl is optionally substituted with hydroxy, C₁₋₆-alkoxy, halo, CF₃, amino or C₁₋₆-alkyl-carbonyl-amino.

In a still further preferred embodiment B represents phenyl or naphthyl, which phenyl or naphthyl is optionally substituted with C₁₋₆-alkoxy, halo, amino or C₁₋₆-alkyl-carbonyl-amino.

In a still further preferred embodiment B represents phenyl or naphthyl, which phenyl or naphthyl is optionally substituted with methoxy, fluoro, chloro, amino or methyl-carbonyl-amino (acetamido).

In a still further preferred embodiment B represents naphthyl, which is optionally substituted with methoxy, fluoro, chloro, amino or methyl-carbonyl-amino (acetamido).

In a still further preferred embodiment B represents phenyl or naphthyl.

In a still further preferred embodiment B represents phenyl.

In a still further preferred embodiment B represents naphthyl.

In a sixth preferred embodiment the carbocyclic or heterocyclic group is selected from the group consisting of phenyl, naphthyl, furanyl, thienyl and pyridinyl, which heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

In a more preferred embodiment carbocyclic or heterocyclic group is selected from the group consisting of phenyl, naphthyl, furanyl, thienyl and pyridinyl, which heterocyclic groups are optionally substituted with C₁₋₆-alkoxy, halo, amino or C₁₋₆-alkyl-carbonyl-amino.

In an even more preferred embodiment the carbocyclic or heterocyclic group is selected from the group consisting of phenyl, naphthyl, furanyl, thienyl and pyridinyl, which heterocyclic groups are optionally substituted with methoxy, fluoro, chloro, amino or methyl-carbonyl-amino (acetamido).

In a most preferred embodiment the diazabicyclic aryl derivative of the invention is
8-Methyl-3-(5-phenyl-[1,3,4]thiadiazol-2-yl)-3,8-diaza-bicyclo[3.2.1]octane;
8-Methyl-3-(5-phenyl-[1,3,4]oxadiazol-2-yl)-3,8-diaza-bicyclo[3.2.1]octane;
8-Methyl-3-(6-phenyl-pyridin-2-yl)-3,8-diaza-bicyclo[3.2.1]octane;
8-Methyl-3-(6-thiophen-2-yl-pyridazin-3-yl)-3,8-diaza-bicyclo[3.2.1]octane;
8-Methyl-3-(6-thiophen-3-yl-pyridazin-3-yl)-3,8-diaza-bicyclo[3.2.1]octane;
3-(6-Furan-2-yl-pyridazin-3-yl)-8-methyl-3,8-diaza-bicyclo[3.2.1]octane;
8-Methyl-3-(6-pyridin-3-yl-pyridazin-3-yl)-3,8-diaza-bicyclo[3.2.1]octane;
3-(6-Furan-3-yl-pyridazin-3-yl)-8-methyl-3,8-diaza-bicyclo[3.2.1]octane;
3-[6-(4-Methoxy-phenyl)-pyridazin-3-yl]-8-methyl-3,8-diaza-bicyclo[3.2.1]octane;
3-[6-(8-Methyl-3,8-diaza-bicyclo[3.2.1]oct-3-yl)-pyridazin-3-yl]-phenylamine;
*N*-{3-[6-(8-Methyl-3,8-diaza-bicyclo[3.2.1]oct-3-yl)-pyridazin-3-yl]-phenyl}-acetamide;
3-[6-(2-Fluoro-phenyl)-pyridazin-3-yl]-8-methyl-3,8-diaza-bicyclo[3.2.1]octane;
3-[6-(4-Fluoro-phenyl)-pyridazin-3-yl]-8-methyl-3,8-diaza-bicyclo[3.2.1]octane;
3-[6-(3-Fluoro-phenyl)-pyridazin-3-yl]-8-methyl-3,8-diaza-bicyclo[3.2.1]octane;
2-[6-(8-Methyl-3,8-diaza-bicyclo[3.2.1]oct-3-yl)-pyridazin-3-yl]-phenylamine;
8-Methyl-3-(5-phenyl-pyridin-2-yl)-3,8-diaza-bicyclo[3.2.1]octane;
3-[6-(2-Methoxy-phenyl)-pyridazin-3-yl]-8-methyl-3,8-diaza-bicyclo[3.2.1]octane;
3-[6-(3-Methoxy-phenyl)-pyridazin-3-yl]-8-methyl-3,8-diaza-bicyclo[3.2.1]octane;
*N*-{2-[6-(8-Methyl-3,8-diaza-bicyclo[3.2.1]oct-3-yl)-pyridazin-3-yl]-phenyl}-acetamide;
3-[6-(3-Chloro-phenyl)-pyridazin-3-yl]-8-methyl-3,8-diaza-bicyclo[3.2.1]octane;
3-[6-(4-Chloro-phenyl)-pyridazin-3-yl]-8-methyl-3,8-diaza-bicyclo[3.2.1]octane;
3-[6-(2-Chloro-phenyl)-pyridazin-3-yl]-8-methyl-3,8-diaza-bicyclo[3.2.1]octane; or
3-[6-(6-Methoxy-naphthalen-2-yl)-pyridazin-3-yl]-8-methyl-3,8-diaza-bicyclo[3.2.1]octane;
or an enantiomer or a mixture of its enantiomers, or a pharmaceutically acceptable salt thereof.

Any combination of two or more of the embodiments described herein is considered within the scope of the present invention.

### Definition of Substituents

In the context of this invention halo represents a fluorine, a chlorine, a bromine or an iodine atom. Thus, a trihalomethyl group represents e.g. a trifluoromethyl group, a trichloromethyl group and similar trihalo-substituted methyl groups.

In the context of this invention an alkyl group designates a univalent saturated, straight or branched hydrocarbon chain. The hydrocarbon chain preferably contain of from one to eighteen carbon atoms (C₁₋₁₈-alkyl); more preferred of from one to six carbon atoms (C₁₋₆-alkyl; lower alkyl), including pentyl, isopentyl, neopentyl, tertiary pentyl, hexyl and isohexyl. In a preferred embodiment alkyl represents a C₁₋₄-alkyl group, including butyl, isobutyl, secondary butyl, and tertiary butyl. In another preferred embodiment of this invention alkyl represents a C₁₋₃-alkyl group, which may in particular be methyl, ethyl, propyl or isopropyl.

In the context of this invention a cycloalkyl group designates a cyclic alkyl group, preferably containing of from three to seven carbon atoms (C₃₋₇-cycloalkyl), including cyclopropyl, cyclobutyl, cyclopentyl, cyclohexyl and cycloheptyl.

In the context of this invention a cycloalkyl-alkyl group designates a cycloalkyl group as defined above, which cycloalkyl group is substituted on an alkyl group as also defined above. Examples of preferred cycloalkyl-alkyl groups of the invention include cyclopropylmethyl and cyclopropylethyl.

In the context of this invention an alkoxy group designates an "alkyl-O-" group, wherein alkyl is as defined above. Examples of preferred alkoxy groups of the invention include methoxy and ethoxy.

In the context of this invention a cyanoalkyl group designates an "-alkyl-CN" group, wherein alkyl is as defined above.

In the context of this invention an alkyl-carbonyl-amino group designates an "alkyl-CO-NH-" group, wherein alkyl is as defined above. Preferred alkyl-carbonyl-amino groups of the invention include acetamido.

In the context of this invention an aromatic monocyclic or bicyclic carbocyclic group designates a monocyclic or polycyclic aromatic hydrocarbon group. Examples of preferred aryl groups of the invention include phenyl, indenyl, naphthyl, azulenyl, fluorenyl, and anthracenyl.

In the context of this invention an aromatic monocyclic or bicyclic heterocyclic group is a mono- or bicyclic compound, which holds one or more heteroatoms in its ring structure. The term "bi- and poly-heterocyclic groups" includes benzo-fused five- and six-membered heterocyclic rings containing one or more heteroatoms. Preferred heteroatoms include nitrogen (N), oxygen (O), and sulphur (S).

In the context of this invention a 5-6 membered aromatic monocyclic heterocyclic designates a 5- or 6-membered heteroaryl, which holds one or more heteroatoms in its ring structure. Preferred heteroatoms include nitrogen (N), oxygen (O), and sulphur (S).

Preferred 5 membered heteroaryl groups of the invention include furanyl, in particular furan-2- or 3-yl; thienyl, in particular thien-2- or 3-yl; pyrrolyl (azolyl), in particular pyrrol-2- or 3-yl; oxazolyl, in particular oxazol-2, 4- or 5-yl; thiazolyl, in particular thiazol-2, 4- or 5-yl; isoxazolyl, in particular isoxazol-3, 4- or 5-yl; isothiazolyl, in particular isothiazol-3-, 4- or 5-yl; oxadiazolyl, in particular 1,2,3-oxadiazol-4,5-diyl or 1,3,4-oxadiazol-2,5-diyl and thiadiazolyl, in particular 1,2,3-thiadiazol-4- or 5-yl, or 1,3,4-thiadiazol-2-yl.

More preferred 5 membered heteroaryl groups of the invention include oxadiazolyl, in particular 1,2,3-oxadiazol-4,5-diyl or 1,3,4-oxadiazol-2,5-diyl and thiadiazolyl, in particular 1,2,3-thiadiazol-4- or 5-yl, or 1,3,4-thiadiazol-2-yl.

Preferred 6 membered heteroaryl groups of the invention include pyridinyl, in particular pyrid-2-, 3- or 4-yl; and pyrazinyl, in particular pyrazin-2- or 3-yl.

Preferred bicyclic heteroaryl groups of the invention include indolyl, in particular indol-2-, 5- or 6-yl; benzo[b]furanyl, in particular benzofuran-2-, 5- or 6-yl; benzo[b]thienyl, in particular benzothien-2-, 5- or 6-yl; and benzothiazolyl, in particular benzothiazol-2-, 5- or 6-yl.

### Pharmaceutically Acceptable Salts

The diazabicyclic aryl derivative of the invention may be provided in any form suitable for the intended administration. Suitable forms include pharmaceutically (i.e. physiologically) acceptable salts, and pre- or prodrug forms of the chemical compound of the invention.

Examples of pharmaceutically acceptable addition salts include, without limitation, the non-toxic inorganic and organic acid addition salts such as the hydrochloride, the hydrobromide, the nitrate, the perchlorate, the phosphate, the sulphate, the formate, the acetate, the aconate, the ascorbate, the benzenesulphonate, the benzoate, the cinnamate, the citrate, the embonate, the enantate, the fumarate, the glutamate, the glycolate, the lactate, the maleate, the malonate, the mandelate, the methanesulphonate, the naphthalene-2-sulphonate derived, the phthalate, the salicylate, the sorbate, the stearate, the succinate, the tartrate, the toluene-p-sulphonate, and the like. Such salts may be formed by procedures well known and described in the art.

Metal salts of a chemical compound of the invention include alkali metal salts, such as the sodium salt of a chemical compound of the invention containing a carboxy group.

In the context of this invention the "onium salts" of N-containing compounds are also contemplated as pharmaceutically acceptable salts. Preferred "onium salts" include the alkyl-onium salts, the cycloalkyl-onium salts, and the cycloalkylalkyl-onium salts.

### Steric Isomers

The chemical compounds of the present invention may exist in (+) and (-) forms as well as in racemic forms. The racemates of these isomers and the individual isomers themselves are within the scope of the present invention.

Racemic forms can be resolved into the optical antipodes by known methods and techniques. One way of separating the diastereomeric salts is by use of an optically active acid, and liberating the optically active amine compound by treatment with a base. Another method for resolving racemates into the optical antipodes is based upon chromatography on an optical active matrix. Racemic compounds of the present invention can thus be resolved into their optical antipodes, e.g., by fractional crystallisation of d- or I- (tartrates, mandelates, or camphorsulphonate) salts for example.

The chemical compounds of the present invention may also be resolved by the formation of diastereomeric amides by reaction of the chemical compounds of the present invention with an optically active activated carboxylic acid such as that derived from (+) or (-) phenylalanine, (+) or (-) phenylglycine, (+) or (-) camphanic acid or by the formation of diastereomeric carbamates by reaction of the chemical compound of the present invention with an optically active chloroformate or the like.

Additional methods for the resolving the optical isomers are known in the art. Such methods include those described by Jaques J, Collet A, & Wilen S in "Enantiomers, Racemates, and Resolutions", John Wiley and Sons, New York (1981).

Optical active compounds can also be prepared from optical active starting materials.

### Methods of Producing Diazabicyclic Aryl Derivatives

The diazabicyclic aryl derivative of the invention may be prepared by conventional methods for chemical synthesis, e.g. those described in the working examples. The starting materials for the processes described in the present application are known or may readily be prepared by conventional methods from commercially available chemicals.

Also one compound of the invention can be converted to another compound of the invention using conventional methods.

The end products of the reactions described herein may be isolated by conventional techniques, e.g. by extraction, crystallisation, distillation, chromatography, etc.

### Biological Activity

The present invention is devoted to the provision novel ligands and modulators of the nicotinic receptors, which ligands and modulators are useful for the treatment of diseases or disorders related to the cholinergic receptors, and in particular the nicotinic acetylcholine receptor (nAChR). Preferred compounds of the invention show a pronounced nicotinic acetylcholine α7 receptor subtype selectivity.

The compounds of the present invention may in particular be agonists, partial agonists, antagonists and/or allosteric modulators of the nicotinic acetylcholine receptor.

Due to their pharmacological profile the compounds of the invention may be useful for the treatment of diseases or conditions as diverse as CNS related diseases, PNS related diseases, diseases related to smooth muscle contraction, endocrine disorders, diseases related to neuro-degeneration, diseases related to inflammation, pain, and withdrawal symptoms caused by the termination of abuse of chemical substances.

In a preferred embodiment the compounds of the invention are used for the treatment of diseases, disorders, or conditions relating to the central nervous system. Such diseases or disorders includes anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, peripheral neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In another preferred embodiment the compounds of the invention may be useful for the treatment of diseases, disorders, or conditions associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In yet another preferred embodiment the compounds of the invention may be useful for the treatment of endocrine disorders, such as thyrotoxicosis, pheochromocytoma, hypertension and arrhythmias.

In still another preferred embodiment the compounds of the invention may be useful for the treatment of neurodegenerative disorders, including transient anoxia and induced neuro-degeneration.

In even another preferred embodiment the compounds of the invention may be useful for the treatment of inflammatory diseases, disorders, or conditions, including inflammatory skin disorders such as acne and rosacea, Crohn's disease, inflammatory bowel disease, ulcerative colitis, and diarrhoea.

In still another preferred embodiment the compounds of the invention may be useful for the treatment of mild, moderate or even severe pain of acute, chronic or recurrent character, pain caused by migraine, postoperative pain, phantom limb pain, inflammatory pain, chronic headache, central pain, neuropathic pain, pain related to diabetic neuropathy, to postherpetic neuralgia, or to peripheral nerve injury.

In a further preferred embodiment the compounds of the invention may be useful for the treatment of diabetic neuropathy, schizophrenia, cognitive or attentional deficits related to schizophrenia, or depression.

Finally the compounds of the invention may be useful for the treatment of withdrawal symptoms caused by termination of use of addictive substances. Such addictive substances include nicotine-containing products such as tobacco, opioids such as heroin, cocaine and morphine, benzodiazepines and benzodiazepine-like drugs, and alcohol. Withdrawal from addictive substances is in general a traumatic experience characterised by anxiety and frustration, anger, anxiety, difficulties in concentrating, restlessness, decreased heart rate and increased appetite and weight gain.

In this context "treatment" covers treatment, prevention, prophylactics and alleviation of withdrawal symptoms and abstinence as well as treatment resulting in a voluntary diminished intake of the addictive substance.

In another aspect, the compounds of the invention are used as diagnostic agents, e.g. for the identification and localisation of nicotinic receptors in various tissues.

### Pharmaceutical Compositions

In another aspect the invention provides novel pharmaceutical compositions comprising a therapeutically effective amount of diazabicyclic aryl derivative of the invention.

While a chemical compound of the invention for use in therapy may be administered in the form of the raw chemical compound, it is preferred to introduce the active ingredient, optionally in the form of a physiologically acceptable salt, in a pharmaceutical composition together with one or more adjuvants, excipients, carriers, buffers, diluents, and/or other customary pharmaceutical auxiliaries.

In a preferred embodiment, the invention provides pharmaceutical compositions comprising the diazabicyclic aryl derivative of the invention, or a pharmaceutically acceptable salt or derivative thereof, together with one or more pharmaceutically acceptable carriers therefore, and, optionally, other therapeutic and/or prophylactic ingredients, know and used in the art. The carrier(s) must be "acceptable" in the sense of being compatible with the other ingredients of the formulation and not harmful to the recipient thereof.

The pharmaceutical composition of the invention may be administered by any convenient route, which suits the desired therapy. Preferred routes of administration include oral administration, in particular in tablet, in capsule, in dragé, in powder, or in liquid form, and parenteral administration, in particular cutaneous, subcutaneous, intramuscular, or intravenous injection. The pharmaceutical composition of the invention can be manufactured by any skilled person by use of standard methods and conventional techniques appropriate to the desired formulation. When desired, compositions adapted to give sustained release of the active ingredient may be employed.

Further details on techniques for formulation and administration may be found in the latest edition of Remington's Pharmaceutical Sciences (Maack Publishing Co., Easton, PA).

The actual dosage depends on the nature and severity of the disease being treated, and is within the discretion of the physician, and may be varied by titration of the dosage to the particular circumstances of this invention to produce the desired therapeutic effect. However, it is presently contemplated that pharmaceutical compositions containing of from about 0.1 to about 500 mg of active ingredient per individual dose, preferably of from about 1 to about 100 mg, most preferred of from about 1 to about 10 mg, are suitable for therapeutic treatments.

The active ingredient may be administered in one or several doses per day. A satisfactory result can, in certain instances, be obtained at a dosage as low as 0.1 µg/kg i.v. and 1 µg/kg p.o. The upper limit of the dosage range is presently considered to be about 10 mg/kg i.v. and 100 mg/kg p.o. Preferred ranges are from about 0.1 µg/kg to about 10 mg/kg/day i.v., and from about 1 µg/kg to about 100 mg/kg/day p.o.

### Methods of Therapy

The diazabicyclic aryl derivatives of the present invention are valuable nicotinic and monoamine receptor modulators, and therefore useful for the treatment of a range of ailments involving cholinergic dysfunction as well as a range of disorders responsive to the action of nAChR modulators.

In another aspect the invention provides the diazabicyclic aryl derivatives of the present invention for use in a method for the treatment, prevention or alleviation of a disease or a disorder or a condition of a living animal body, including a human, which disease, disorder or condition is responsive to modulation of cholinergic receptors and/or monoamine receptors, and which method comprises administering to such a living animal body, including a human, in need thereof an effective amount of a diazabicyclic aryl derivative of the invention.

In a preferred embodiment, the disease, disorder or condition relates to the central nervous system.

In a preferred embodiment, the disease, disorder or condition is anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, peripheral neuropathy, autism, dyslexia, tardive dyskinesia, hyperkinesia, epilepsy, bulimia, post-traumatic syndrome, social phobia, sleeping disorders, pseudodementia, Ganser's syndrome, pre-menstrual syndrome, late luteal phase syndrome, chronic fatigue syndrome, mutism, trichotillomania, and jet-lag.

In a another preferred embodiment, the disease, disorder or condition are associated with smooth muscle contractions, including convulsive disorders, angina pectoris, premature labour, convulsions, diarrhoea, asthma, epilepsy, tardive dyskinesia, hyperkinesia, premature ejaculation, and erectile difficulty.

In a third preferred embodiment, the disease, disorder or condition is related to the endocrine system, such as thyrotoxicosis, pheochromocytoma, hypertension and arrhythmias.

In a fourth preferred embodiment, the disease, disorder or condition is a neurodegenerative disorders, including transient anoxia and induced neuro-degeneration.

In a sixth preferred embodiment, the disease, disorder or condition is an inflammatory disorder, including inflammatory skin disorders such as acne and rosacea, Crohn's disease, inflammatory bowel disease, ulcerative colitis, and diarrhoea.

In a seventh preferred embodiment, the disease, disorder or condition is mild, moderate or even severe pain of acute, chronic or recurrent character, as well as pain caused by migraine, postoperative pain, and phantom limb pain.

In an eighth preferred embodiment, the disease, disorder or condition is associated with withdrawal symptoms caused by termination of use of addictive substances, including nicotine-containing products such as tobacco, opioids such as heroin, cocaine and morphine, benzodiazepines and benzodiazepine-like drugs, and alcohol.

It is at present contemplated that suitable dosage ranges are 0.1 to 1000 milligrams daily, 10-500 milligrams daily, and especially 30-100 milligrams daily, dependent as usual upon the exact mode of administration, form in which administered, the indication toward which the administration is directed, the subject involved and the body weight of the subject involved, and further the preference and experience of the physician or veterinarian in charge.

A satisfactory result can, in certain instances, be obtained at a dosage as low as 0.005 mg/kg i.v. and 0.01 mg/kg p.o. The upper limit of the dosage range is about 10 mg/kg i.v. and 100 mg/kg p.o. Preferred ranges are from about 0.001 to about 1 mg/kg i.v. and from about 0.1 to about 10 mg/kg p.o.

### EXAMPLES

The invention is further illustrated with reference to the following examples, which are not intended to be in any way limiting to the scope of the invention as claimed.

### Example 1

### Preparatory Example

All reactions involving air sensitive reagents or intermediates were performed under nitrogen and in anhydrous solvents. Magnesium sulphate was used as drying agent in the workup-procedures and solvents were evaporated under reduced pressure.

### Diethyl cis-1-methylpyrrolidine-25-dicarboxylate (Intermediate compound)

Diethyl *mezo*-2,5-dibromoadipate (101.7 g, 0.283 mol) was dissolved by heating under argon in THF (400 ml) and then cooled to 0°C. To the obtained solution a pre-cooled solution of methylamine (27.3 g, 0.88 mol) in THF (150 ml) was added and the mixture was stirred at room temperature for 18 hours. The separated crystalline material was filtered off, the filtrate concentrated and the residue chromatographied on a silica gel column (10 cm long) with hexane-ethyl acetate 4:1 as eluent to afford 58.9 g (91 %).

¹H NMR (300 MHz, CDCl₃): δ 1.15 (t, 6H); 1.9-2.0 (m, 4H); 2.38 (s, 3H); 2.99 (m, 2H); 4.07 (q, 4H). ¹³C NMR (75 MHz, CDCl₃): δ 13.98; 27.68; 40.82; 60.39; 67.93; 68.06; 172.32.

### 3-Benzyl-8-methyl-3,8-diazabicyclo[3.2.1]octane-2,4-dione (Intermediate compound)

To a solution of diethyl *cis*-1-methylpyrrolidine-2,5-dicarboxylate (74.8 g, 0.383 mol) in xylene (150 ml) benzylamine (41.0 g, 0.383 mol) was added and the mixture heated to reflux for 16 hours. Xylene was removed at reduced pressure and the residue was heated at 220°C for 18 hours. The obtained crude product was distilled by portions (30-40 g) on Büchi oven for distillation at 180°C and 0.1 mbar, and the first fraction collected (after about 1 hour). The combined first fractions were crystallized from a mixture of hexane and ethyl acetate 1:1 to yield 30.6 (34%).
¹H NMR (300 MHz, CDCl₃): δ 1.88 (m, 2H); 2.34 (m, 2H); 2.42 (s, 3H); 3.80 (dd, 2H); 4.88 (s, 2H); 7.2-7.4 (m, 5H). ¹³C NMR (75 MHz, CDCl₃): 26.69; 35.82; 41.26; 65.72; 127.42; 128.36; 128.62; 136.91; 173.26.

### 3-Benzyl-8-methyl-3,8-diazabicyclo[3.2.1]octane (Intermediate compound)

To a solution of 3-benzyl-8-methyl-3,8-diazabicyclo[3.2.1]octane-2,4-dione (28.3 g, 0.116 mol) in 200 ml of absolute dioxane LiAlH₄ (7.6 g, 0.2 mol) was added and the mixture boiled under argon for 18 hours. Then a mixture of water (7.5 ml) and dioxane (40 ml) was added drop-wise to the reaction mixture. The suspension was mixed for 20 minutes and filtered trough a dense glass filter. The filtrate was evaporated and the residue was distilled on Büchi oven for distillation at 120°C and 0.1 mbar. Yield 17.6 g (70%).
¹H NMR (300 MHz, CDCl₃): δ 1.7-1.9 (m, 4H); 2.18 (s, 3H); 2.25 (d, 2H); 2.48 (dd, 2H); 2.95 (m, 2H); 3.39 (s, 2H); 7.1-7.3 (m, 5H).

### 8-Methyl-3,8-diazabicyclo[3.2.1]octane (Intermediate compound)

To a degassed by argon solution of 3-benzyl-8-methyl-3,8-diazabicyclo-[3.2.1]octane (17.6 g, 0.08 mol) in methanol (50 ml), 10% Pd/C (1.0 g) was added and hydrogen passed into reaction mixture for 24 hours. The catalyst was filtered off, the filtrate evaporated and the residue distilled on Büchi oven for distillation at 100°C and 0.1 mbar: Yield 8.5 g (85%).
¹H NMR (300 MHz, CDCl₃): δ 1.6 (m, 2H); 1.86 (s, 1 H); 1.9-2,0 (m, 2H); 2,17 (s, 3H); 2.53 (m, 2H); 2.9-3.0 (m, 4H). ¹³C NMR (75 MHz, CDCl₃): δ 24.73; 41.72; 52.10; 62.08.

### References related to preparation of the intermediate compounds

Cignarella G & Nathansohn G; Gazz. Chim. Ital. 1960 90 1495;
Blackman SW & Baltzly R; J. Org. Chem. 1960 2750; and
Cignarella G, Nathansohn G &, Occelli E; J. Org. Chem. 1960 2747.

### 2-Chloro-5-phenyl-1,3,4-thiadiazole (Intermediate compound)

2-Amino-5-phenyl-1,3,4-thiadiazole sulfate (25.12 g, 142 mmol) was stirred in concentrated hydrochloric acid (300 ml) at 0°C. Sodium nitrite (12.7 g, 184 mmol) was added during a period of 10 minutes. The reaction mixture was stirred at 50°C for 15 hours. The hydrochloric acid was evaporated. Aqueous sodium hydroxide (4M, 250 ml) was added and the precipitate was filtered. Chromatography on silica gel with ethyl acetate as solvent gave a pure product. Yield 15.5 g (56%).

### Method A

### 8-Methyl-3-(6-phenyl-pyridazin-3-yl)-3,8-diaza-bicyclo[3.2.1]octane free base

### (Compound A1)

A mixture of 8-methyl-3,8-diaza-bicyclo[3.2.1]octane (2.0 g, 15.8 mmol) and 3-chloro-6-phenylpyridazine (3.0 g, 15.8 mmol) was stirred at 120°C for 2.5 hours. Aqueous sodium hydroxide (1 M, 50 ml) was added and the mixture was extracted twice with dichloromethane (2 x 50 ml). Chromatography on silica gel with dichloromethane, 10% methanol and 1 % aqueous ammonia as solvent gave the title compound as a crystalline solid. Yield 1.5 g (68%). Mp. 151 °C.

### 8-Methyl-3-(5-phenyl-[1,3,4]thiadiazol-2-yl)-3,8-diaza-bicyclo[3.2.1]octane fumaric acid salt (Compound A2)

Was prepared according to Method A from 8-methyl-3,8-diaza-bicyclo[3.2.1]octane and 2-Chloro-5-phenyl-1,3,4-thiadiazole. The corresponding salt was obtained by addition of a diethyl ether and methanol mixture (9:1) saturated with fumaric acid. Mp. 192-200°C.

### 2-Mercaptobenzyl-5-phenyl-1,3,4-oxadiazole (Intermediate compound)

Benzylbromide (16.8 ml, 141 mmol) was added over a time period of 10 minutes to a mixture of 5-phenyl-1,3,4-oxadiazole-2-thiol (25.2 g, 141 mmol), triethylamine (19.7 ml, 141 mmol) and ethanol (250 ml) at room temperature. The mixture was allowed to stir at room temperature for 3 hours. Aqueous sodium hydroxide (1 M, 250 ml) was added and the mixture was extracted twice with dichloromethane (2 x 200 ml). Chromatography on silica gel with dichloromethane, 10% methanol and 1% aqueous ammonia as solvent gave the title compound as an oil. Yield 34.2 g (90%).

### Method B

### 8-Methyl-3-(5-phenyl-[1,3,4]oxadiazol-2-yl)-3,8-diaza-bicyclo[3.2.1]octane fumaric acid

### salt (Compound B1)

A mixture of 8-methyl-3,8-diaza-bicyclo[3.2.1]octane (0.28 g, 2.23 mmol) and 2-mercaptobenzyl-5-phenyl-1,3,4-oxadiazole (0.60 g, 2.23 mmol) was stirred at 120°C for 2.5 hours. Aqueous sodium hydroxide (1M, 20 ml) was added and the mixture was extracted twice with dichloromethane (2 x 20 ml). Chromatography on silica gel with dichloromethane, 10% methanol and 1% aqueous ammonia as solvent gave the compound as a crystalline solid. The corresponding salt was obtained by addition of a diethyl ether and methanol mixture (9:1) saturated with fumaric acid. Yield 0.16 g (19%). Mp. 170-191°C.

### Method C

### 3-(6-Bromo-pyridazin-3-yl)-8-methyl-3,8-diaza-bicyclo[3.2.1]octane free base

### (Intermediate compound)

A mixture of 3,6-dibromoyridazine (13.0 g, 54.6 mmol), 8-methyl-3,8-diaza-bicyclo[3.2.1]octane (6.9 g, 54.6 mmol) and dioxane (100 ml) was stirred at room temperature for 15 hours followed by 70°C for 12 hours. Aqueous sodium hydroxide (1 M, 100 ml) was added and the mixture was extracted twice with dichloromethane (2 x 100 ml). Chromatography on silica gel with dichloromethane, 10% methanol and 1% aqueous ammonia as solvent gave the compound as a crystalline solid. Mp. 118.5-119.5°C.

### 3-(6-Bromo-pyridin-2-yl)-8-methyl-3,8-diaza-bicyclo[3.2.1]octane fumaric acid salt (Intermediate compound)

Was prepared according to Method C. Mp. 182.3°C.

### Method D

### 8-Methyl-3-(6-phenyl-pyridin-2-yl)-3,8-diaza-bicyclo[3.2.1]octane fumaric acid salt

### (Compound D1)

A mixture of 3-(6-bromo-pyridin-2-yl)-8-methyl-3,8-diaza-bicyclo[3.2.1]-octane (1.0 g, 3.45 mmol), phenyltrimethyltin (1.71 g, 7.09 mmol), PdCl₂(PPh₃)₂ (124 mg, 0.177 mmol) and dioxane (20 ml) was stirred at reflux for 15 hours. Aqueous sodium hydroxide (50 ml, 1M) was added and the mixture was extracted twice with dichloromethane (2 x 50 ml). Chromatography on silica gel with dichloromethane, 10% methanol and 1% aqueous ammonia as solvent gave the compound as a crystalline solid. The corresponding salt was obtained by addition of a diethyl ether and methanol mixture (9:1) saturated with fumaric acid. Mp. 159-160°C.

### Method E

### 8-Methyl-3-(6-thiophen-2-yl-pyridazin-3-yl)-3,8-diaza-bicyclo[3,2,1]octane free base

### (Compound E1)

A mixture of 3-(6-bromo-pyridazin-3-yl)-8-methyl-3,8-diaza-bicyclo[3.2.1]-octane (0.50 g, 1.77 mmol), thiopheneboronic acid (339 mg, 2.65 mmol), palladacycle (33 mg, 0.035 mmol), 1,3-propandiol (403 mg, 5.30 mmol), potassium carbonate (732 mg, 5.3 mmol), water (2.65 ml) and 1,2-dimethoxyethane (25 ml) was stirred at reflux for 15 hours. Aqueous sodium hydroxide (1 M, 20 ml) was added and the mixture was extracted twice with dichloromethane (2 x 20 ml). Chromatography on silica gel with dichloromethane, 10% methanol and 1% aqueous ammonia as solvent gave the compound as a crystalline solid. Mp. 156°C.

### 8-Methyl-3-(6-thiophen-3-yl-pyridazin-3-yl)-3,8-diaza-bicyclo[3.2.1]octane free base

### (Compound E2)

Was prepared according to Method E. Mp. 172-173°C.

### 3-(6-Furan-2-yl-pyridazin-3-yl)-8-methyl-3,8-diaza-bicyclo[3.2.1]octane free base

### (Compound E3)

Was prepared according to Method E. Mp. 144°C.

### 8-Methyl-3-(6-pyridin-3-yl-pyridazin-3-yl)-3,8-diaza-bicyclo[3.2.1]octane free base

### (Compound E4)

Was prepared according to Method E. Mp. 131-133°C.

### 3-(6-Furan-3-yl-pyridazin-3-yl)-8-methyl-3,8-diaza-bicyclo[3.2.1]octane free base

### (Compound E5)

Was prepared according to Method E. Mp. 152°C.

### 3-[6-(4-Methoxy-phenyl)-pyridazin-3-yl]-8-methyl-3,8-diaza-bicyclo[3.2.1]octane free

### base (Compound E6)

Was prepared according to Method E. Mp. 154-155°C.

### 3-[6-(8-Methyl-3,8-diaza-bicyclo[3.2.1]oct-3-yl)-pyridazin-3-yl]-phenylamine free base

### (Compound E7)

Was prepared according to Method E. Mp. 164°C.

### N-{3-[6-(8-Methyl-3,8-diaza-bicyclo[3.2.1]oct-3-yl)-pyridazin-3-yl]-phenyl}-acetamide

### free base (Compound E8)

Was prepared according to Method E. Mp. 74°C.

### 3-[6-(2-Fluoro-phenyl)-pyridazin-3-yl]-8-methyl-3,8-diaza-bicyclo[3.2.1]octane free

### base (Compound E9)

Was prepared according to Method E. Mp. 152°C.

### 3-[6-(4-Fluoro-phenyl)-pyridazin-3-yl]-8-methyl-3,8-diaza-bicyclo[3.2.1]octane free

### base (Compound E10)

Was prepared according to Method E. Mp. 164-165°C.

### 3-[6-(3-Fluoro-phenyl)-pyridazin-3-yl]-8-methyl-3,8-diaza-bicyclo[3.2.1]octane free

### base (Compound E11)

Was prepared according to Method E. Mp. 149-150°C.

### 2-[6-(8-Methyl-3,8-diaza-bicyclo[3.2.1]oct-3-yl)-pyridazin-3-yl]-phenylamine fumaric

### acid salt (Compound E12)

Was prepared according to Method E. Mp. 186-195°C.

### 8-Methyl-3-(5-phenyl-pyridin-2-yl)-3,8-diaza-bicyclo[3.2.1]octane free base

### (Compound E13)

Was prepared according to Method E from 3-(5-bromo-pyridin-2-yl)-8-methyl-3,8-diaza-bicyclo[3.2.1]octane. Mp. 110.5°C.

### 3-[6-(2-Methoxy-phenyl)-pyridazin-3-yl]-8-methyl-3,8-diaza-bicyclo[3.2.1]octane free

### base (Compound E14)

Was prepared according to Method E. Mp. 170-172°C.

### 3-[6-(3-Methoxy-phenyl)-pyridazin-3-yl]-8-methyl-3,8-diaza-bicyclo[3.2.1]octane free

### base (Compound E15)

Was prepared according to Method E. Mp. 92-97°C.

### N-{2-[6-(8-Methyl-3,8-diaza-bicyclo[3.2.1]oct-3-yl)-pyridazin-3-yl]-phenyl}-acetamide

### fumaric acid salt (Compound E16)

Was prepared according to Method E. Mp. 219-222°C.

### 3-[6-(3-Chloro-phenyl)-pyridazin-3-yl]-8-methyl-3,8-diaza-bicyclo[3.2.1]octane free

### base (Compound E17)

Was prepared according to Method E. Mp. 159°C.

### 3-[6-(4-Chloro-phenyl)-pyridazin-3-yl]-8-methyl-3,8-diaza-bicyclo[3.2.1]octane free

### base (Compound E18)

Was prepared according to Method E. Mp. 180°C.

### 3-[6-(2-Chloro-phenyl)-pyridazin-3-yl]-8-methyl-3,8-diaza-bicyclo[3.2.1]octane fumaric

### acid salt (Compound E19)

Was prepared according to Method E. Mp. 192-200°C.

### 3-[6-(6-Methoxy-naphthalen-2-yl)-pyridazin-3-yl]-8-methyl-3,8-diaza-bicyclo[3.2.1]octane fumaric acid salt (Compound E20)

Was prepared according to Method E. Mp. 242°C.

### Example 2

### In vitro Inhibition of ³H-α-Bungarotoxine Binding in Rat Brain

In this example the affinity of the compounds of the invention for binding to α₇-subtype of nicotinic receptors is determined.

α-Bungarotoxine is a peptide isolated from the venom of the Elapidae snake *Bungarus multicinctus.* It has high affinity for neuronal and neuromuscular nicotinic receptors, where it acts as a potent antagonist.

³H-α-Bungarotoxine labels nicotinic acetylcholine receptors formed by the α₇ subunit isoform found in brain and the α₁ isoform in the neuromuscular junction.

### Tissue preparation

Preparations are performed at 0-4°C. Cerebral cortices from male Wistar rats (150-250 g) are homogenised for 10 seconds in 15 ml of 20 mM Hepes buffer containing 118 mM NaCl, 4.8 mM KCI, 1.2 mM MgSO₄ and 2.5 mM CaCl₂ (pH 7.5) using an Ultra-Turrax homogeniser. The tissue suspension is subjected to centrifugation at 27,000 x g for 10 minutes. The supernatant is discarded and the pellet is washed twice by centrifugation at 27,000 x g for 10 minutes in 20 ml of fresh buffer, and the final pellet is then re-suspended in fresh buffer containing 0.01 % BSA (35 ml per g of original tissue) and used for binding assays.

### Assay

Aliquots of 500 µl of homogenate are added to 25 µl of test solution and 25 µl of ³H-α-bungarotoxine (2 nM, final concentration) and mixed and incubated for 2 hours at 37°C. Non-specific binding is determined using (-)-nicotine (1 mM, final concentration). After incubation, the samples are added 5 ml of ice-cold Hepes buffer containing 0.05% PEI and poured directly onto Whatman GF/C glass fibre filters (presoaked in 0.1 % PEI for at least 6 hours) under suction, and immediately washed with 2 x 5 ml ice-cold buffer.

The amount of radioactivity on the filters is determined by conventional liquid scintillation counting. Specific binding is total binding minus non-specific binding.

The test value is given as an IC₅₀ (the concentration of the test substance which inhibits the specific binding of ³H-α-bungarotoxin by 50%).

Initially compounds E1, E2, E3, E5, E7, E8, E9 and E11 were subjected to this assay and they all showed results at the sub-micro-molar (i.e. < 1 µM) level.

## Claims

1. A diazabicyclic aryl derivative represented by Formula I 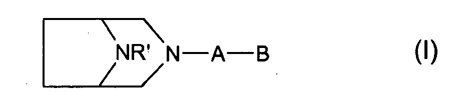 any of its enantiomers or any mixture of its enantiomers, or a pharmaceutically acceptable salt thereof, wherein
R' represents hydrogen or C₁₋₆-alkyl ;
A represents an aromatic monocyclic group selected from phenyl, furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl; pyrazinyl and triazinyl; and
B represents an aromatic monocyclic or bicyclic carbocyclic or heterocyclic group, which carbocyclic or heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

2. The diazabicyclic aryl derivative of claim 1, wherein R' represents hydrogen or C₁₋₆-alkyl.

3. The diazabicyclic aryl derivative of claim 2, wherein R' represents C₁₋₆-alkyl.

4. The diazabicyclic aryl derivative of any one of claims 1-3, wherein A represents an aromatic monocyclic group selected from phenyl, furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl and triazinyl.

5. The diazabicyclic aryl derivative of claim 4, wherein A represents an aromatic heterocyclic group selected from furanyl, thienyl, selenophenyl, pyrrolyl, oxazolyl, thiazolyl, imidazolyl, pyrazolyl, isoxazolyl, isothiazolyl, oxadiazolyl, triazolyl, thiadiazolyl, pyridinyl, pyridazinyl, pyrimidinyl, pyrazinyl and triazinyl.

6. The diazabicyclic aryl derivative of claim 5, wherein A represents oxazolyl, thiazolyl, oxadiazolyl, thiadiazolyl, pyridinyl, pyrimidinyl, pyrazinyl or pyridazinyl.

7. The diazabicyclic aryl derivative of any one of claims 1-6, wherein B represents an aromatic monocyclic or bicyclic carbocyclic or heterocyclic group, which carbocyclic or heterocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

8. The diazabicyclic aryl derivative of claim 7, wherein B represents phenyl, naphthyl, furanyl, thienyl or pyridinyl, which phenyl, naphthyl, furanyl, thienyl and pyridinyl are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

9. The diazabicyclic aryl derivative of claim 7, wherein B represents phenyl, naphthyl, furanyl, thienyl or pyridinyl, which phenyl, naphthyl, furanyl, thienyl and pyridinyl are optionally substituted with C₁₋₆-alkoxy, halo, amino or C₁₋₆-alkyl-carbonyl-amino.

10. The diazabicyclic aryl derivative of claim 7, wherein B represents phenyl or naphthyl, which carbocyclic groups are optionally substituted one or more times with substituents selected from the group consisting of C₁₋₆-alkyl, C₃₋₇-cycloalkyl, C₃₋₇-cycloalkyl-C₁₋₆-alkyl, hydroxy, C₁₋₆-alkoxy, cyano-C₁₋₆-alkyl, halo, trihalo-C₁₋₆-alkyl, trihalo-C₁₋₆-alkoxy, cyano, nitro, amino and C₁₋₆-alkyl-carbonyl-amino.

11. The diazabicyclic aryl derivative of claim 9, wherein B represents phenyl or naphthyl.

12. The diazabicyclic aryl derivative of claim 1, which is 8-Methyl-3-(5-phenyl-[1,3,4]thiadiazol-2-yl)-3,8-diaza-bicyclo[3.2.1]octane;
8-Methyl-3-(5-phenyl-[1,3,4]oxadiazol-2-yl)-3,8-diaza-bicyclo[3.2.1]octane;
8-Methyl-3-(6-phenyl-pyridin-2-yl)-3,8-diaza-bicyclo[3.2.1]octane;
8-Methyl-3-(6-thiophen-2-yl-pyridazin-3-yl)-3,8-diaza-bicyclo[3.2.1]octane;
8-Methyl-3-(6-thiophen-3-yl-pyridazin-3-yl)-3,8-diaza-bicyclo[3.2.1]octane;
3-(6-Furan-2-yl-pyridazin-3-yl)-8-methyl-3,8-diaza-bicyclo[3.2.1]octane;
8-Methyl-3-(6-pyridin-3-yl-pyridazin-3-yl)-3,8-diaza-bicyclo[3.2.1]octane;
3-(6-Furan-3-yl-pyridazin-3-yl)-8-methyl-3,8-diaza-bicyclo[3.2.1]octane;
3-[6-(4-Methoxy-phenyl)-pyridazin-3-yl]-8-methyl-3,8-diaza-bicyclo[3.2.1]octane;
3-[6-(8-Methyl-3,8-diaza-bicyclo[3.2.1]oct-3-yl)-pyridazin-3-yl]-phenylamine;
*N*-{3-[6-(8-Methyl-3,8-diaza-bicyclo[3.2.1]oct-3-yl)-pyridazin-3-yl]-phenyl}-acetamide;
3-[6-(2-Fluoro-phenyl)-pyridazin-3-yl]-8-methyl-3,8-diaza-bicyclo[3.2.1 ]octane;
3-[6-(4-Fluoro-phenyl)-pyridazin-3-yl]-8-methyl-3,8-diaza-bicyclo[3.2.1]octane;
3-[6-(3-Fluoro-phenyl)-pyridazin-3-yl]-8-methyl-3,8-diaza-bicyclo[3.2.1]octane;
2-[6-(8-Methyl-3,8-diaza-bicyclo[3.2.1]oct-3-yl)-pyridazin-3-yl]-phenylamine;
8-Methyl-3-(5-phenyl-pyridin-2-yl)-3,8-diaza-bicyclo[3.2.1]octane; 3-[6-(2-Methoxy-phenyl)-pyridazin-3-yl]-8-methyl-3,8-diaza-bicyclo[3.2.1]octane;
3-[6-(3-Methoxy-phenyl)-pyridazin-3-yl]-8-methyl-3,8-diaza-bicyclo[3.2.1]octane;
*N*-{2-[6-(8-Methyl-3,8-diaza-bicyclo[3.2.1]oct-3-yl)-pyridazin-3-yl]-phenyl}-acetamide;
3-[6-(3-Chloro-phenyl)-pyridazin-3-yl]-8-methyl-3,8-diaza-bicyclo[3.2.1]octane;
3-[6-(4-Chloro-phenyl)-pyridazin-3-yl]-8-methyl-3,8-diaza-bicyclo[3.2.1]octane;
3-[6-(2-Chloro-phenyl)-pyridazin-3-yl]-8-methyl-3,8-diaza-bicyclo[3.2.1]octane; or
3-[6-(6-Methoxy-naphthalen-2-yl)-pyridazin-3-yl]-8-methyl-3,8-diaza-bicyclo[3.2.1]octane;
or an enantiomer or a mixture of its enantiomers, or a pharmaceutically acceptable salt thereof.

13. A pharmaceutical composition comprising a therapeutically effective amount of a diazabicyclic aryl derivative of any one of claims 1-12, or a pharmaceutically-acceptable addition salt thereof, together with at least one pharmaceutically-acceptable carrier or diluent.

14. Use of a diazabicyclic aryl derivative of any one of claims 1-12, or a pharmaceutically-acceptable addition salt thereof, for the manufacture of a pharmaceutical composition/medicament for the treatment, prevention or alleviation of a disease or a disorder or a condition of a mammal, including a human, which disease, disorder or condition is responsive to modulation of cholinergic receptors and/or monoamine receptors.

15. The use according to claim 14, wherein the disease, disorder or condition relates to the central nervous system.

16. The use according to claim 14, wherein the disease, disorder or condition is anxiety, cognitive disorders, learning deficit, memory deficits and dysfunction, Alzheimer's disease, attention deficit, attention deficit hyperactivity disorder, Parkinson's disease, Huntington's disease, Amyotrophic Lateral Sclerosis, Gilles de la Tourette's syndrome, depression, mania, manic depression, schizophrenia, obsessive compulsive disorders (OCD), panic disorders, eating disorders such as anorexia nervosa, bulimia and obesity, narcolepsy, nociception, AIDS-dementia, senile dementia, diabetic neuropathy, peripheral neuropathy, autism, dyslexia, tardive dyskinesia, hyperkinesia, epilepsy, post-traumatic syndrome, social phobia, sleeping disorders, pseudodementia, Ganser's syndrome, pre-menstrual syndrome, late luteal phase syndrome, chronic fatigue syndrome, mutism, trichotillomania, or jet-lag.

17. The use according to claim 14, wherein the disease, disorder or condition are associated with smooth muscle contractions, convulsive disorders, angina pectoris, premature labour, convulsions, diarrhoea, asthma, epilepsy, tardive dyskinesia, hyperkinesia, premature ejaculation, or erectile difficulty.

18. The use according to claim 14, wherein the disease, disorder or condition is related to the endocrine system, such as thyrotoxicosis, pheochromocytoma, hypertension and arrhythmias.

19. The use according to claim 14, wherein the disease, disorder or condition is a neurodegenerative disorder, transient anoxia or induced neuro-degeneration.

20. The use according to claim 14, wherein the disease, disorder or condition is an inflammatory disorder, an inflammatory skin disorder, acne, rosacea, Crohn's disease, inflammatory bowel disease, ulcerative colitis, or diarrhoea.

21. The use according to claim 14, wherein the disease, disorder or condition is mild, moderate or severe pain, pain of acute, chronic or recurrent character, pain caused by migraine, postoperative pain, phantom limb pain, inflammatory pain, chronic headache, central pain, neuropathic pain, pain related to diabetic neuropathy, to postherpetic neuralgia, or to peripheral nerve injury.

22. The use according to claim 14, wherein the disease, disorder or condition is diabetic neuropathy, schizophrenia, cognitive or attention deficits related to schizophrenia, or depression.

23. The use according to claim 14, wherein the disease, disorder or condition is associated with withdrawal symptoms caused by termination of use of addictive substances, nicotine-containing products, tobacco, heroin, cocaine, morphine, benzodiazepines and benzodiazepine-like drugs, or alcohol.

## Patentansprüche

1. Diazabicyclisches Arylderivat, das durch Formel I wiedergegeben ist 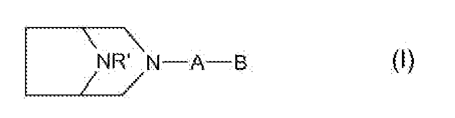 beliebige von seinen Enantiomeren oder eine beliebige Mischung von seinen Enantiomeren, oder ein pharmazeutisch verträgliches Salz davon, wobei
R' Wasserstoff oder C₁₋₆-Alkyl bedeutet;
A eine aromatische monocyclische Gruppe bedeutet, die ausgewählt ist aus Phenyl, Furanyl, Thienyl, Selenophenyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridinyl, Pyridazinyl, Pyrimidinyl, Pyrazinyl und Triazinyl; und
B eine aromatische monocyclische oder bicyclische carbocyclische oder heterocyclische Gruppe bedeutet, wobei die carbocyclischen oder heterocyclischen Gruppen gegebenenfalls einfach oder mehrfach mit Substituenten substituiert sind, die ausgewählt sind aus der Gruppe bestehend aus C₁₋₆-Alkyl, C₃₋₇-Cycloalkyl, C₃₋₇-Cycloalkyl-C₁₋₆-Alkyl, Hydroxy, C₁₋₆-Alkoxy, Cyano-C₁₋₆-alkyl, Halogen, Trihalogen-C₁₋₆-alkyl, Trihalo-gen-C₁₋₆-alkoxy, Cyano, Nitro, Amino und C₁₋₆-Alkyl-carbonyl-amino.

2. Diazabicyclisches Arylderivat nach Anspruch 1, wobei R' Wasserstoff oder C₁₋₆-Alkyl bedeutet.

3. Diazabicyclisches Arylderivat nach Anspruch 2, wobei R' C₁₋₆-Alkyl bedeutet.

4. Diazabicyclisches Arylderivat nach einem der Ansprüche 1-3, wobei A eine aromatische monocyclische Gruppe bedeutet, die ausgewählt ist aus Phenyl, Furanyl, Thienyl, Selenophenyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridinyl, Pyridazinyl, Pyrimidinyl, Pyrazinyl und Triazinyl.

5. Diazabicyclisches Arylderivat nach Anspruch 4, wobei A eine aromatische heterocyclische Gruppe bedeutet, die ausgewählt ist aus Furanyl, Thienyl, Selenophenyl, Pyrrolyl, Oxazolyl, Thiazolyl, Imidazolyl, Pyrazolyl, Isoxazolyl, Isothiazolyl, Oxadiazolyl, Triazolyl, Thiadiazolyl, Pyridinyl, Pyridazinyl, Pyrimidinyl, Pyrazinyl und Triazinyl.

6. Diazabicyclisches Arylderivat nach Anspruch 5, wobei A Oxazolyl, Thiazolyl, Oxadiazolyl, Thiadiazolyl, Pyridinyl, Pyrimidinyl, Pyrazinyl oder Pyridazinyl bedeutet.

7. Diazabicyclisches Arylderivat nach einem der Ansprüche 1-6, wobei B eine aromatische monocyclische oder bicyclische carbocyclische oder heterocyclische Gruppe bedeutet, wobei die carbocyclischen oder heterocyclischen Gruppen gegebenenfalls einfach oder mehrfach mit Substituenten substituiert sind, die ausgewählt sind aus der Gruppe bestehend aus C₁₋₆-Alkyl, C₃₋₇-Cycloalkyl, C₃₋₇-Cycloalkyl-C₁₋₆-Alkyl, Hydroxy, C₁₋₆-Alkoxy, Cyano-C₁₋₆-alkyl, Halogen, Trihalogen-C₁₋₆-alkyl, Trihalogen-C₁₋₆-alkoxy, Cyano, Nitro, Amino und C₁₋₆-Alkyl-carbonyl-amino.

8. Diazabicyclisches Arylderivat nach Anspruch 7, wobei B Phenyl, Naphthyl, Furanyl, Thienyl oder Pyridinyl bedeutet, wobei das Phenyl, Naphthyl, Furanyl, Thienyl und Pyridinyl gegebenenfalls einfach oder mehrfach mit Substituenten substituiert ist, die ausgewählt sind aus der Gruppe bestehend aus C₁₋₆-Alkyl, C₃₋₇-Cycloalkyl, C₃₋₇-Cycloalkyl-C₁₋₆-Alkyl, Hydroxy, C₁₋₆-Alkoxy, Cyano-C₁₋₆-alkyl, Halogen, Trihalogen-C₁₋₆-alkyl, Trihalogen-C₁₋₆-alkoxy, Cyano, Nitro, Amino und C₁₋₆-Alkyl-carbonyl-amino.

9. Diazabicyclisches Arylderivat nach Anspruch 7, wobei B Phenyl, Naphthyl, Furanyl, Thienyl oder Pyridinyl bedeutet, wobei das Phenyl, Naphthyl, Furanyl, Thienyl und Pyridinyl gegebenenfalls mit C₁₋₆-Alkoxy, Halogen, Amino oder C₁₋₆-Alkyl-carbonyl-amino substituiert ist.

10. Diazabicyclisches Arylderivat nach Anspruch 7, wobei B Phenyl oder Naphthyl bedeutet, wobei die carbocyclischen Gruppen gegebenenfalls einfach oder mehrfach mit Substituenten substituiert sind, die ausgewählt sind aus der Gruppe bestehend aus C₁₋₆-Alkyl, C₃₋₇-Cycloalkyl, C₃₋₇-Cycloalkyl-C₁₋₆-alkyl, Hydroxy, C₁₋₆-Alkoxy, Cyano-C₁₋₆-alkyl, Halogen, Trihalogen-C₁₋₆-alkyl, Trihalogen-C₁₋₆-alkoxy, Cyano, Nitro, Amino und C₁₋₆-Alkyl-carbonyl-amino.

11. Diazabicyclisches Arylderivat nach Anspruch 9, wobei B Phenyl oder Naphthyl bedeutet.

12. Diazabicyclisches Arylderivat nach Anspruch 1, welches
8-Methyl-3-(5-phenyl-[1,3,4]thiadiazol-2-yl)-3,8-diaza-bicyclo[3.2.1]octan;
8-Methyl-3-(5-phenyl-[1,3,4]oxadiazol-2-yl)-3,8-diaza-bicyclo[3.2.1]octan;
8-Methyl-3-(6-phenyl-pyridin-2-yl)-3,8-diaza-bicyclo[3.2.1]octan;
8-Methyl-3-(6-thiophen-2-yl-pyridazin-3-yl)-3,8-diaza-bicyclo[3.2.1]octan;
8-Methyl-3-(6-thiophen-3-yl-pyridazin-3-yl)-3,8-diaza-bicyclo[3.2.1]octan;
3-(6-Furan-2-yl-pyridazin-3-yl)-8-methyl-3,8-diaza-bicyclo[3.2.1]octan;
8-Methyl-3-(6-pyridin-3-yl-pyridazin-3-yl)-3,8-diaza-bicyclo[3.2.1]octan;
3-(6-Furan-3-yl-pyridazin-3-yl)-8-methyl-3,8-diaza-bicyclo[3.2.1]octan;
3-[6-(4-Methoxy-phenyl)-pyridazin-3-yl]-8-methyl-3,8-diaza-bicyclo[3.2.1]octan;
3-[6-(8-Methyl-3,8-diaza-bicyclo[3.2.1]oct-3-yl)-pyridazin-3-yl]-phenylamin;
*N*-{3-[6-(8-Methyl-3,8-diaza-bicyclo[3.2.1]oct-3-yl)-pyridazin-3-yl]-phenyl}-acetamid;
3-[6-(2-Fluor-phenyl)-pyridazin-3-yl]-8-methyl-3,8-diaza-bicyclo[3.2.1]octan;
3-[6-(4-Fluor-phenyl)-pyridazin-3-yl]-8-methyl-3,8-diaza-bicyclo[3.2.1]octan;
3-[6-(3-Fluor-phenyl)-pyridazin-3-yl]-8-methyl-3,8-diaza-bicyclo[3.2.1]octan;
2-[6-(8-Methyl-3,8-diaza-bicyclo[3.2.1]oct-3-yl)-pyridazin-3-yl]-phenylamin;
8-Methyl-3-(5-phenyl-pyridin-2-yl)-3,8-diaza-bicyclo[3.2.1]octan;
3-[6-(2-Methoxy-phenyl)-pyridazin-3-yl]-8-methyl-3,8-diaza-bicyclo[3.2.1]octan;
3-[6-(3-Methoxy-phenyl)-pyridazin-3-yl]-8-methyl-3,8-diaza-bicyclo[3.2.1]octan;
*N*-{2-[6-(8-Methyl-3,8-diaza-bicyclo[3.2.1]oct-3-yl)-pyridazin-3-yl]-phenyl}-acetamid;
3-[6-(3-Chlor-phenyl)-pyridazin-3-yl]-8-methyl-3,8-diaza-bicyclo[3.2.1]octan;
3-[6-(4-Chlor-phenyl)-pyridazin-3-yl]-8-methyl-3,8-diaza-bicyclo[3.2.1]octan;
3-[6-(2-Chlor-phenyl)-pyridazin-3-yl]-8-methyl-3,8-diaza-bicyclo[3.2.1]octan; oder
3-[6-(6-Methoxy-naphthalen-2-yl)-pyridazin-3-yl]-8-methyl-3,8-diaza-bicyclo[3.2.1]octan ist;
oder ein Enantiomer oder eine Mischung von seinen Enantiomeren, oder ein pharmazeutisch verträgliches Salz davon.

13. Pharmazeutische Zusammensetzung umfassend eine therapeutisch wirksame Menge von einem diazabicyclischen Arylderivat nach einem der Ansprüche 1-12, oder einem pharmazeutisch verträglichen Additionssalz davon, zusammen mit wenigstens einem pharmazeutisch verträglichen Träger oder Verdünnungsmittel.

14. Verwendung eines diazabicyclischen Arylderivats nach einem der Ansprüche 1-12, oder eines pharmazeutisch verträglichen Additionssalzes davon, für die Herstellung einer pharmazeutischen Zusammensetzung/eines Medikaments für die Behandlung, Verhütung oder Linderung einer Krankheit oder einer Störung oder eines Zustands eines Säugers, einschließlich eines Menschen, wobei die Krankheit, die Störung oder der Zustand auf die Modulation von cholinergen Rezeptoren und/oder Monoaminrezeptoren anspricht.

15. Verwendung nach Anspruch 14, wobei sich die Krankheit, die Störung oder der Zustand auf das Zentralnervensystem bezieht.

16. Verwendung nach Anspruch 14, wobei es sich bei der Krankheit, der Störung oder dem Zustand um Angst, kognitive Störungen, ein Lerndefizit, Gedächtnisdefizite und eine Gedächtnisdysfunktion, die Alzheimer-Krankheit, ein Aufmerksamkeitsdefizit, eine Aufmerksamkeitsdefizit-Hyperaktivitätsstörung, die Parkinson-Krankheit, Chorea Huntington, amyotrophe Lateralsklerose, das Gilles de la Tourette-Syndrom, eine Depression, eine Manie, eine manische Depression, eine Schizophrenie, Zwangsstörungen (OCD), Panikstörungen, Essstörungen wie Anorexia nervosa, Bulimie und Fettleibigkeit, Narkolepsie, Nozizeption, AIDS-Demenz, senile Demenz, diabetische Neuropathie, periphere Neuropathie, Autismus, Dyslexie, tardive Dyskinesie, Hyperkinesie, Epilepsie, ein posttraumatisches Syndrom, eine Sozialphobie, Schlafstörungen, Pseudodemenz, das Ganser-Syndrom, das prämenstruelle Syndrom, das Syndrom der späten Lutealphase, das chronische Ermüdungssyndrom, Mutismus, Trichotillomanie oder Jetlag handelt.

17. Verwendung nach Anspruch 14, wobei die Krankheit, die Störung oder der Zustand mit der Kontraktion eines glatten Muskels, konvulsiven Störungen, Angina pectoris, einer Frühgeburt, Krämpfen, Diarrhö, Asthma, Epilepsie, tardiver Dyskinesie, Hyperkinesie, vorzeitiger Ejakulation oder Erektionsschwierigkeiten zusammenhängen.

18. Verwendung nach Anspruch 14, wobei die Krankheit, die Störung oder der Zustand mit dem endokrinen System zusammenhängt, wie etwa Thyreotoxikose, Phäochromozytom, Hochdruck und Arrhythmien.

19. Verwendung nach Anspruch 14, wobei es sich bei der Krankheit, der Störung oder dem Zustand um eine neurodegenerative Störung, vorübergehende Anoxie oder induzierte Neurodegeneration handelt.

20. Verwendung nach Anspruch 14, wobei es sich bei der Krankheit, der Störung oder dem Zustand um eine entzündliche Störung, eine entzündliche Hautstörung, Akne, Rosacea, Morbus Crohn, eine entzündliche Darmerkrankung, Colitis ulcerosa oder Diarrhö handelt.

21. Verwendung nach Anspruch 14, wobei es sich bei der Krankheit, der Störung oder dem Zustand um milde, mäßige oder starke Schmerzen, Schmerzen von akutem, chronischem oder wiederkehrendem Charakter, durch Migräne verursachte Schmerzen, postoperative Schmerzen, Phantomschmerzen, entzündliche Schmerzen, chronische Kopfschmerzen, zentrale Schmerzen, neuropathische Schmerzen, Schmerzen in Verbindung mit einer diabetischen Neuropathie, postherpetischen Neuralgie oder peripheren Nervenverletzung handelt.

22. Verwendung nach Anspruch 14, wobei es sich bei der Krankheit, der Störung oder dem Zustand um diabetische Neuropathie, Schizophrenie, kognitive oder Aufmerksamkeitsdefizite in Verbindung mit Schizophrenie oder eine Depression handelt.

23. Verwendung nach Anspruch 14, wobei die Krankheit, die Störung oder der Zustand mit Entzugssymptomen zusammenhängt, die durch die Beendigung des Gebrauchs von süchtigmachenden Substanzen, nikotinhaltigen Produkten, Tabak, Heroin, Kokain, Morphin, Benzodiazepinen und benzodiazepinartigen Drogen bzw. Arzneimitteln oder Alkohol verursacht sind.

## Revendications

1. Dérivé arylé diazabicyclique représenté par la formule I 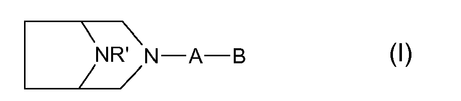 l'un quelconque de ses énantiomères ou tout mélange de ses énantiomères, ou sel pharmaceutiquement acceptable de celui-ci, dans laquelle
R' représente un hydrogène ou un alkyle en C₁₋₆ ;
A représente un groupe monocyclique aromatique choisi parmi les phényle, furanyle, thiényle, sélénophényle, pyrrolyle, oxazolyle, thiazolyle, imidazolyle, pyrazolyle, isoxazolyle, isothiazolyle, oxadiazolyle, triazolyle, thiadiazolyle, pyridinyle, pyridazinyle, pyrimidinyle, pyrazinyle et triazinyle ; et
B représente un groupe carbocyclique ou hétérocyclique aromatique monocyclique ou bicyclique, lesquels groupes carbocycliques ou hétérocycliques sont facultativement substitués une ou plusieurs fois par des substituants choisis dans le groupe constitué par un alkyle en C₁₋₆, un cycloalkyle en C₃₋₇, un cycloalkyl en C₃₋₇-alkyle en C₁₋₆, un hydroxy, un alcoxy en C₁₋₆, un cyano-alkyle en C₁₋₆, un halogéno, un trihalogéno-alkyle en C₁₋₆, un trihalogéno-alcoxy en C₁₋₆, un cyano, un nitro, un amino et un alkyl en C₁₋₆-carbonyl-amino.

2. Dérivé arylé diazabicyclique selon la revendication 1, dans lequel R' représente un hydrogène ou un alkyle en C₁₋₆.

3. Dérivé arylé diazabicyclique selon la revendication 2, dans lequel R' représente un alkyle en C₁₋₆.

4. Dérivé arylé diazabicyclique selon l'une quelconque des revendications 1 à 3, dans lequel A représente un groupe monocyclique aromatique choisi parmi les phényle, furanyle, thiényle, sélénophényle, pyrrolyle, oxazolyle, thiazolyle, imidazolyle, pyrazolyle, isoxazolyle, isothiazolyle, oxadiazolyle, triazolyle, thiadiazolyle, pyridinyle, pyridazinyle, pyrimidinyle, pyrazinyle et triazinyle.

5. Dérivé arylé diazabicyclique selon la revendication 4, dans lequel A représente un groupe hétérocyclique aromatique choisi parmi les furanyle, thiényle, sélénophényle, pyrrolyle, oxazolyle, thiazolyle, imidazolyle, pyrazolyle, isoxazolyle, isothiazolyle, oxadiazolyle, triazolyle, thiadiazolyle, pyridinyle, pyridazinyle, pyrimidinyle, pyrazinyle et triazinyle.

6. Dérivé arylé diazabicyclique selon la revendication 5, dans lequel A représente un oxazolyle, un thiazolyle, un oxadiazolyle, un thiadiazolyle, un pyridinyle, un pyrimidinyle, un pyrazinyle ou un pyridazinyle.

7. Dérivé arylé diazabicyclique selon l'une quelconque des revendications 1 à 6, dans lequel B représente un groupe carbocyclique ou hétérocyclique aromatique monocyclique ou bicyclique, lesquels groupes carbocycliques ou hétérocycliques sont facultativement substitués une ou plusieurs fois par des substituants choisis dans le groupe constitué par un alkyle en C₁₋₆, un cycloalkyle en C₃₋₇, un cycloalkyl en C₃₋₇-alkyle en C₁₋₆, un hydroxy, un alcoxy en C₁₋₆, un cyano-alkyle en C₁₋₆, un halogéno, un trihalogéno-alkyle en C₁₋₆, un trihalogéno-alcoxy en C₁₋₆, un cyano, un nitro, un amino et un alkyl en C₁₋₆-carbonyl-amino.

8. Dérivé arylé diazabicyclique selon la revendication 7, dans lequel B représente un phényle, un naphtyle, un furanyle, un thiényle ou un pyridinyle, lesquels phényle, naphtyle, furanyle, thiényle et pyridinyle sont facultativement substitués une ou plusieurs fois par des substituants choisis dans le groupe constitué par un alkyle en C₁₋₆, un cycloalkyle en C₃₋₇, un cycloalkyl en C₃₋₇-alkyle en C₁₋₆, un hydroxy, un alcoxy en C₁₋₆, un cyano-alkyle en C₁₋₆, un halogéno, un trihalogéno-alkyle en C₁₋₆, un trihalogéno-alcoxy en C₁₋₆, un cyano, un nitro, un amino et un alkyl en C₁₋₆-carbonyl-amino.

9. Dérivé arylé diazabicyclique selon la revendication 7, dans lequel B représente un phényle, un naphtyle, un furanyle, un thiényle ou un pyridinyle, lesquels phényle, naphtyle, furanyle, thiényle et pyridinyle sont facultativement substitués par un alcoxy en C₁₋₆, un halogéno, un amino ou un alkyl en C₁₋₆-carbonyl-amino.

10. Dérivé arylé diazabicyclique selon la revendication 7, dans lequel B représente un phényle ou un naphtyle, lesquels groupes carbocycliques sont substitués une ou plusieurs fois par des substituants choisis dans le groupe constitué par un alkyle en C₁₋₆, un cycloalkyle en C₃₋₇, un cycloalkyl en C₃₋₇-alkyle en C₁₋₆, un hydroxy, un alcoxy en C₁₋₆, un cyano-alkyle en C₁₋₆, un halogéno, un trihalogéno-alkyle en C₁₋₆, un trihalogéno-alcoxy en C₁₋₆, un cyano, un nitro, un amino et un alkyl en C₁₋₆-carbonyl-amino.

11. Dérivé arylé diazabicyclique selon la revendication 9, dans lequel B représente un phényle ou un naphtyle.

12. Dérivé arylé diazabicyclique selon la revendication 1, qui est
le 8-méthyl-3-(5-phényl-[1,3,4]thiadiazol-2-yl)-3,8-diaza-bicyclo[3.2.1]octane ;
le 8-méthyl-3-(5-phényl-[1,3,4]oxadiazol-2-yl)-3,8-diaza-bicyclo[3.2.1]octane ;
le 8-méthyl-3-(6-phényl-pyridin-2-yl)-3,8-diaza-bicyclo[3.2.1]octane ;
le 8-méthyl-3-(6-thiophén-2-yl-pyridazin-3-yl)-3,8-diaza-bicyclo[3.2.1]octane ;
le 8-méthyl-3-(6-thiophén-3-yl-pyridazin-3-yl)-3,8-diaza-bicyclo[3.2.1]octane ;
le 3-(6-furan-2-yl-pyridazin-3-yl)-8-méthyl-3,8-diaza-bicyclo[3.2.1]octane ;
le 8-méthyl-3-(6-pyridin-3-yl-pyridazin-3-yl)-3,8-diaza-bicyclo[3.2.1]octane ;
le 3-(6-furan-3-yl-pyridazin-3-yl)-8-méthyl-3,8-diaza-bicyclo[3.2.1]octane ;
le 3-[6-(4-méthoxy-phényl)-pyridazin-3-yl]-8-méthyl-3,8-diaza-bicyclo[3.2.1]octane ;
la 3-[6-(8-méthyl-3,8-diaza-bicyclo[3.2.1]oct-3-yl)-pyridazin-3-yl]-phénylamine ;
le N-{3-[6-(8-méthyl-3,8-diaza-bicyclo[3.2.1]oct-3-yl)-pyridazin-3-yl]-phényl}-acétamide ;
le 3-[6-(2-fluoro-phényl)-pyridazin-3-yl]-8-méthyl-3,8-diaza-bicyclo[3.2.1]octane ;
le 3-[6-(4-fluoro-phényl)-pyridazin-3-yl]-8-méthyl-3,8-diaza-bicyclo[3.2.1]octane ;
le 3-[6-(3-fluoro-phényl)-pyridazin-3-yl]-8-méthyl-3,8-diaza-bicyclo[3.2.1]octane ;
la 2-[6-(8-méthyl-3,8-diaza-bicyclo[3.2.1]oct-3-yl)-pyridazin-3-yl]-phénylamine ;
le 8-méthyl-3-(5-phényl-pyridin-2-yl)-3,8-diaza-bicyclo[3.2.1]octane ;
le 3-[6-(2-méthoxy-phényl)-pyridazin-3-yl]-8-méthyl-3,8-diaza-bicyclo[3.2.1]octane ;
le 3-[6-(3-méthoxy-phényl)-pyridazin-3-yl]-8-méthyl-3,8-diaza-bicyclo[3.2.1]octane ;
le N-{2-[6-(8-méthyl-3,8-diaza-bicyclo[3.2.1]oct-3-yl)-pyridazin-3-yl]-phényl}-acétamide ;
le 3-[6-(3-chloro-phényl)-pyridazin-3-yl]-8-méthyl-3,8-diaza-bicyclo[3.2.1]octane ;
le 3-[6-(4-chloro-phényl)-pyridazin-3-yl]-8-méthyl-3,8-diaza-bicyclo[3.2.1]octane ;
le 3-[6-(2-chloro-phényl)-pyridazin-3-yl]-8-méthyl-3,8-diaza-bicyclo[3.2.1]octane ; ou
le 3-[6-(6-méthoxy-naphthalén-2-yl)-pyridazin-3-yl]-8-méthyl-3,8-diaza-bicyclo[3.2.1]octane ;
ou un énantiomère ou un mélange de ses énantiomères, ou un sel pharmaceutiquement acceptable de celui-ci.

13. Composition pharmaceutique comprenant une quantité thérapeutiquement efficace d'un dérivé arylé diazabicyclique selon l'une quelconque des revendications 1 à 12, ou d'un sel d'addition pharmaceutiquement acceptable de celui-ci, conjointement avec au moins un support ou diluant pharmaceutiquement acceptable.

14. Utilisation d'un dérivé arylé diazabicyclique selon l'une quelconque des revendications 1 à 12, ou d'un sel d'addition pharmaceutiquement acceptable de celui-ci, pour la fabrication d'une composition pharmaceutique/médicament pour le traitement, la prévention ou l'atténuation d'une maladie ou d'un trouble ou d'un état d'un mammifère, incluant un être humain, laquelle maladie, lequel trouble ou lequel état réagit à une modulation des récepteurs cholinergiques et/ou des récepteurs monoamines.

15. Utilisation selon la revendication 14, dans laquelle la maladie, le trouble ou l'état se rapporte au système nerveux central.

16. Utilisation selon la revendication 14, dans laquelle la maladie, le trouble ou l'état est une anxiété, des troubles cognitifs, une déficience d'apprentissage, des déficiences et un dysfonctionnement de la mémoire, une maladie d'Alzheimer, une déficience de l'attention, un trouble d'hyperactivité avec déficience de l'attention, une maladie de Parkinson, une maladie de Huntington, une sclérose latérale amyotrophique, un syndrome de Gilles de la Tourette, une dépression, une manie, une manie dépression, une schizophrénie, des troubles obsessivo-compulsifs (TOC), des troubles paniques, des troubles de l'alimentation tels qu'une anorexie mentale, une boulimie et une obésité, une narcolepsie, une nociception, une démence liée au SIDA, une démence sénile, une neuropathie diabétique, une neuropathie périphérique, un autisme, une dyslexie, une dyskinésie tardive, une hyperkinésie, une épilepsie, un syndrome post-traumatique, une phobie sociale, des troubles du sommeil, une pseudo-démence, un syndrome de Ganser, un syndrome prémenstruel, un syndrome de phase lutéale tardive, un syndrome de fatigue chronique, un mutisme, une trichotillomanie ou un décalage horaire.

17. Utilisation selon la revendication 14, dans laquelle la maladie, le trouble ou l'état est associé(e) à des contractions des muscles lisses, des troubles convulsifs, une angine de poitrine, un travail prématuré, des convulsions, une diarrhée, un asthme, une épilepsie, une dyskinésie tardive, une hyperkinésie, une éjaculation prématurée ou une difficulté d'érection.

18. Utilisation selon la revendication 14, dans laquelle la maladie, le trouble ou l'état est lié(e) au système endocrinien, comme une thyro-toxicose, un phéochromocytome, une hypertension et des arythmies.

19. Utilisation selon la revendication 14, dans laquelle la maladie, le trouble ou l'état est un trouble neurodégénératif, une anoxie transitoire ou une neurodégénérescence induite.

20. Utilisation selon la revendication 14, dans laquelle la maladie, le trouble ou l'état est un trouble inflammatoire, un trouble cutané inflammatoire, une acné, une rosacée, une maladie de Chron, une maladie intestinale inflammatoire, une recto-colite hémorragique ou une diarrhée.

21. Utilisation selon la revendication 14, dans laquelle la maladie, le trouble ou l'état est une douleur légère, modérée ou sévère, une douleur de caractère aigu, chronique ou récurrent, une douleur provoquée par une migraine, une douleur postopératoire, une douleur de membre fantôme, une douleur inflammatoire, une céphalée chronique, une douleur centrale, une douleur neuropathique, une douleur liée à une neuropathie diabétique, à une névralgie post-zostérienne ou à une lésion de nerf périphérique.

22. Utilisation selon la revendication 14, dans laquelle la maladie, le trouble ou l'état est une neuropathie diabétique, une schizophrénie, des déficiences cognitives ou de l'attention liées à une schizophrénie, ou une dépression.

23. Utilisation selon la revendication 14, dans laquelle la maladie, le trouble ou l'état est associé(e) à des symptômes de sevrage provoqués par l'arrêt d'utilisation de substances toxicomanogènes, de produits contenant de la nicotine, de tabac, d'héroïne, de cocaïne, de morphine, de benzodiazépines et de substances médicamenteuses de type benzodiazépine, ou d'alcool.
